# EUROPEAN PATENT APPLICATION

(11) **EP 4 248 884 A1**
(43) Date of publication of application: **27.09.2023**
(21) Application number: 21894001.3
(22) Date of filing: 19.11.2021
(51) Int. Cl.: A61B 17/11

(54) **ANASTOMOTIC STOMA PROTECTION DEVICE**

(30) Priority: 20.11.2020 CN 202011311177; 20.11.2020 CN 202022714232 U; 20.11.2020 CN 202011311144; 20.11.2020 CN 202022714162 U
(71) Applicant: Touchstone International Medical Science Co., Ltd., Suzhou Jiangsu 215123 (CN)
(72) Inventor: SHAN, Teng, Suzhou, Jiangsu 215123 (CN); CHEN, Wangdong, Suzhou, Jiangsu 215123 (CN); CAO, Yuanyang, Suzhou, Jiangsu 215123 (CN)
(74) Representative: Studio Torta S.p.A.
(86) International application number: PCT/CN2021/131647
(87) International publication number: WO 2022/105854

(57) **Abstract**

An anastomotic stoma protection device, comprising: a protection sleeve (1) located at the position in a tubular tissue corresponding to an anastomotic stoma; a first fixing assembly disposed on the outer wall of the tubular tissue, the first fixing assembly comprising an electromagnetic generation component; power supply assembly electrically connected to the electromagnetic generation assembly, and used for supplying power for the electromagnetic generation assembly; and a second fixing assembly disposed on the inner surface of the protection sleeve (1), the second fixing assembly and the first fixing assembly are relatively fixed by means of magnetic adsorption. The protection sleeve (1) protecting the anastomotic stoma is fixed at a desired position by means of magnetic adsorption between the electromagnetic generation component and the internal fixing assembly, and thus does not hinder normal peristalsis of the tubular tissue and provides the tubular tissue with space for circumferential expansion and contraction, thereby ensuring normal blood supply near the anastomotic stoma.

## Description

### TECHNICAL FIELD

The present disclosure relates to medical instruments' technology, more particularly, to an anastomosis protection device.

### BACKGROUND

After anastomoses surgery of intestine is operated, at least one anastomotic stoma is formed. In order to avoid a tension applied by feces on the anastomotic stoma or avoid contamination and infection to the anastomotic stoma caused by feces, the anastomotic stoma needs to be protected.

The existing method of protecting the anastomotic stoma generally includes: setting a tubular sleeve inside the intestine, the tubular sleeve covering an inner surface of the intestine at a position corresponding to the anastomotic stoma, using the tubular sleeve to guide the feces out of the human body. This method effectively protects the anastomotic stoma, and further allows for removing the tubular sleeve without a need for secondary surgery after physiological tissues at the anastomotic stoma are healed. In order to better fix the tubular sleeve at a desired position, a fixing strap with a fixed diameter surrounding the intestine needs to be installed on the outside of the tubular sleeve. However, this fixed strap inevitably applies a certain continuous pressure on the intestine, thereby hindering normal intestinal peristalsis, and may cause problems such as poor blood supply around the anastomotic stoma due to the continuous pressure from the fixed strap.

### SUMMARY

To solve the problems in the prior art, the present disclosure provides an anastomosis protection device, to fix the protective sleeve for protecting the anastomotic stoma at a desired position through the magnetic attraction between at least one electromagnetic member and an internal fixing assembly.

In the present disclosure, an anastomosis protection device is provided, including:
a protective sleeve located inside a tubular tissue and positioned correspondingly to an anastomotic stoma;
a first fixing assembly provided on an outer wall of the tubular tissue, wherein the first fixing assembly comprises at least one electromagnetic member;
a power supply assembly electrically connected to the electromagnetic member, used for supplying power to the electromagnetic member, wherein the electromagnetic member is configured to become magnetic when powered on; and
a second fixing assembly provided on an inner surface of the protective sleeve, wherein the second fixing assembly and the electromagnetic member of the first fixing assembly re relatively fixed by means of magnetic attraction.

In some embodiments, the electromagnetic member comprises at least one electromagnet.

In some embodiments, the first fixing assembly comprises a plurality of electromagnetic members arranged at intervals, and the plurality of electromagnetic members are arranged sequentially in a circumferential direction of the tubular tissue.

In some embodiments, the second fixing assembly comprises a plurality of internal fixing members arranged at intervals, and the internal fixing members are magnetic members or attractable by magnets.

In some embodiments, the power supply assembly comprises a power supply wire, which surrounds the outer wall of the tubular tissue and is electrically connected to the electromagnetic members.

In some embodiments, the power supply wire comprises a plurality of wire accommodating portions and a plurality of wire connecting portions, the wire accommodating portions surround outer sides of the electromagnetic members, and the wire connecting portion is connected between two adjacent wire accommodating portions, the wire accommodating portions and the wire connecting portions together form a circular structure surrounding the outer wall of the tubular tissue.

In some embodiments, the power supply wire further comprises an extension portion, one end of the extension portion is connected to the wire connection portions, and the other end of the extension portion extends out of human body.

In some embodiments, an outer side of the electromagnetic member is provided with a first mounting groove in a circumferential direction of the electromagnetic member, and the wire accommodating portion of the power supply wire is embedded in the first mounting groove; or, an inner side of the wire accommodating portion of the power supply wire is provided with a first mounting groove in a circumferential direction of the wire accommodating portion, and the electromagnetic member is embedded in the first mounting groove.

In some embodiments, a width of the wire accommodating portion is greater than a width of the wire connecting portion, and/or a thickness of the wire accommodating portion is greater than a thickness of the wire connecting portion.

In some embodiments, the power supply wire is elastic.

In some embodiments, an elasticity of the wire connecting portion is greater than an elasticity of the wire accommodating portion.

In some embodiments, the first fixing assembly further comprises a first connector surrounding the outer wall of the tubular tissue, the first connector comprises a plurality of first accommodating portions being configured to accommodate the electromagnetic members and a plurality of first connecting portions, the first connecting portion is connected between two adjacent first accommodating portions, the first accommodating portions surround outer sides of the wire accommodating portions.

In some embodiments, the power supply wire comprises a first connecting structure, the power supply wire has a state of being connected at the first connecting structure to surround the outer wall of the tubular tissue, and another state of being disconnected at the first connecting structure to be separated from the outer wall of the tubular tissue.

In some embodiments, the first fixing assembly further comprises a first connector surrounding the outer wall of the tubular tissue, and the first connector is connected to the electromagnetic member.

In some embodiments, the first connector comprises a second connecting structure, the first connector has a state of being connected at the second connecting structure to surround the outer wall of the tubular tissue, and another state of being disconnected at the second connecting structure to be separated from the outer wall of the tubular tissue.

In some embodiments, the first fixing component comprises a plurality of internal fixing members and a second connector being configured to accommodate the internal fixing members, and the second connector is located on the inner surface of the protective sleeve.

In some embodiments, at least a part of the first connector, the second connector, and/or the internal fixing members are made of bioabsorbable materials.

In some embodiments, the first fixing assembly further comprises a first connector being configured to accommodate the electromagnetic member; and/or the second fixing assembly comprises at least one internal fixing member and a second connector being configured to accommodate the internal fixing member;
at least a part of a circumference of the electromagnetic member is provided with a mounting groove, and an inner circumference of the first connector is embedded in the mounting groove, or at least a part of an inner circumference of the first connector is provided with at least one mounting groove, and the electromagnetic member is embedded in the mounting groove;
at least a part of a circumference of the internal fixing member is provided with a mounting groove, and an inner circumference of the second connector is embedded in the mounting groove, or at least a part of an inner circumference of the second connector is provided with at least one mounting groove, and the internal fixing member is embedded in the mounting groove.

In some embodiments, an end of the protective sleeve located at an upstream side of the anastomotic stoma is provided with a support portion, the support portion is expandable and contractable in a radial direction of the protective sleeve, and the first fixing assembly is arranged between the anastomotic stoma and the support portion.

In some embodiments, the first fixing assembly and the second fixing assembly are arranged at an upstream side of the anastomotic stoma; or
the anastomosis protection device comprises two first fixing assemblies and two second fixing assemblies, one of the first fixing assemblies and one of the second fixing assemblies are located at the upstream side of the anastomotic stoma, and the other first fixing assembly and the other second fixing assembly are located at the downstream side of the anastomotic stoma.

In some embodiments, an output current of the power supply assembly is regulable.

In some embodiments, the power supply assembly comprises an input power source, a current regulating circuit, a control element, and a power supply wire, the input power source supplies power to the electromagnetic member sequentially through the current regulating circuit and the power supply wire, and the control element is used for adjusting a value of an output current of the current regulating circuit.

In some embodiments, the current regulating circuit comprises a transformer, and a number of turns on a secondary winding of the transformer is adjustable, or
the current regulating circuit comprises a voltage dividing circuit or a current dividing circuit, and at least one current dividing branch in the voltage dividing circuit or the current dividing circuit comprises a variable resistor.

In some embodiments, the device further comprises a signal acquisition assembly, wherein the signal acquisition assembly comprises at least one pressure sensor arranged on a side of the first fixing assembly facing the tubular tissue and/or a side of the second fixing assembly facing the protective sleeve.

In some embodiments, the device further comprises a signal processing assembly used for determining a power supply current value based on a predetermined power supply current change period, and/or determining the power supply current value based on detection data of the pressure sensor;
the signal processing assembly is further used for sending a driving signal comprising the power supply current value to the power supply assembly, and the power supply assembly is configured to control a value of an output current based on the power supply current value.

In some embodiments, there are a plurality of pressor sensors, the power supply assembly comprises a plurality of electromagnetic driving modules provided correspondingly to the pressure sensors, and the signal processing assembly is configured to determine a power supply current value of the electromagnetic driving module based on the detection data of the corresponding pressure sensor, and send a driving signal comprising the power supply current value to the corresponding electromagnetic driving module.

In some embodiments, the signal processing assembly is further used for comparing the detection data of the pressure sensors with a preset reference value, and determining whether to alarm based on comparison results; or
the signal processing assembly is further used for comparing the detection data of a plurality of pressure sensors with each other, and determining whether to alarm based on comparison results; or the signal processing assembly is further used for comparing the detection data of the pressure sensors with a preset reference value, or comparing the detection data of a plurality of pressure sensors with each other, and determining whether to generate the power supply current value based on comparison results, and send the driving signal comprising the power supply current value to the electromagnetic driving modules.

In some embodiments, the device further comprises a display module for displaying the detection data of the pressure sensor and/or a value of the output current of the power supply assembly.

In some embodiments, the signal processing assembly transmits data with the pressure sensor and the power supply assembly through signal wires or wireless communication, respectively.

The anastomosis protection device has the following advantages.

The device protects the inner surface of the anastomotic stoma through a protective sleeve, so contents in the tubular tissue won't contaminate the anastomotic stoma when passing through. The protective sleeve is fixed at the desired position through magnetic attraction between the electromagnetic member of the first fixing assembly and the second fixing assembly. In this device, only the magnetic attraction position between the electromagnetic member and the second fixing assembly is relatively fixed, which reduces the impact on the normal peristalsis of the tubular tissue and provides a circumferential extension and retraction space for the tubular tissue, ensuring normal blood supply around the anastomotic stoma. By providing the electromagnetic member and the power supply assembly, the magnetic field can be flexibly produced or eliminated by controlling on-off of the power supply, and a magnitude of magnetism in the electromagnetic member can be flexibly adjusted by adjusting a value of the power supply current and a number of turns on the electromagnetic member. The anastomosis protection device of the present disclosure can be applied to the intestine or other tubular tissues in the human body, such as tubular tissues at other positions of the digestive tract.

### BRIEF DESCRIPTION OF THE DRAWINGS

Embodiments of the present disclosure will now be described, by way of example only, with reference to the accompanying schematic drawings. Apparently, the following figures are only exemplary. For the skilled in the art, other figures can also be gotten according to the following figures without creative work.
FIG. 1 is a structural schematic view of an anastomosis protection device applied to intestinal tissue according to a first embodiment of the present disclosure;
FIG. 2 is a structural schematic view of the anastomosis protection device according to the first embodiment of the present disclosure;
FIG. 3 is an exploded view of the anastomosis protection device according to the first embodiment of the present disclosure;
FIG. 4 is a schematic view showing cooperation between a first fixing assembly and a power supply assembly according to the first embodiment of the present disclosure;
FIG. 5 is a schematic view showing a first connector being disconnected according to the first embodiment of the present disclosure;
FIG. 6 is a schematic view showing a power supply wire being disconnected according to the first embodiment of the present disclosure;
FIG. 7 is a front view of the power supply wire according to the first embodiment of the present disclosure;
FIG. 8 is a side view of the power supply wire according to the first embodiment of the present disclosure;
FIG. 9 is a front view of the first connector according to the first embodiment of the present disclosure;
FIG. 10 is a side view of the first connector according to the first embodiment of the present disclosure;
FIG. 11 is a structural schematic view of an anastomosis protection device according to a second embodiment of the present disclosure;
FIG. 12 is a structural schematic view of an anastomosis protection device applied to intestinal tissue according to a third embodiment of the present disclosure;
FIG. 13 is a structural schematic view of an anastomosis protection device applied to intestinal tissue according to a fourth embodiment of the present disclosure;
FIG. 14 is a schematic view showing cooperation between internal fixing members and a second connector according to the fourth embodiment of the present disclosure;
FIG. 15 is a front view of the second connector according to the fourth embodiment of the present disclosure;
FIG. 16 is a side view of the second connector according to the fourth embodiment of the present disclosure;
FIG. 17 is a structural schematic view of an anastomosis protection device applied to intestinal tissue according to a fifth embodiment of the present disclosure;
FIGS. 18 is a structural schematic view of an anastomosis protection device applied to intestinal tissue according to a sixth embodiment of the present disclosure;
FIG. 19 is a structural schematic view of an anastomosis protection device according to a seventh embodiment of the present disclosure;
FIG. 20 is an exploded view of the anastomosis protection device according to the seventh embodiment of the present disclosure;
FIG. 21 is a schematic view of a first kind of current adjusting circuit according to the seventh embodiment of the present disclosure;
FIG. 22 is a schematic view of a second kind of current adjusting circuit according to the seventh embodiment of the present disclosure;
FIG. 23 is a schematic view of a third kind of current adjusting circuit according to the seventh embodiment of the present disclosure;
FIG. 24 is a schematic view of a first kind of anastomosis protection device with a signal acquisition assembly and a signal processing assembly according to the seventh embodiment of the present disclosure;
FIG. 25 is a schematic view of a second kind of anastomosis protection device with a signal acquisition assembly and a signal processing assembly according to the seventh embodiment of the present disclosure;
FIG. 26 is a structural schematic view of an anastomosis protection device according to an eighth embodiment of the present disclosure;
FIG. 27 is a structural schematic view of an anastomosis protection device applied to intestinal tissue according to a ninth embodiment of the present disclosure;
FIG. 28 is a structural schematic view of an anastomosis protection device applied to intestinal tissue according to a tenth embodiment of the present disclosure.

### DETAILED DESCRIPTION

The exemplary embodiments will be described more comprehensively referring to the accompanying drawings. However, the exemplary embodiments can be implemented in a plurality of forms and should not be limited to the embodiments described herein. On the contrary, these embodiments are provided to make the present disclosure comprehensive and complete, and comprehensively convey the concept of exemplary embodiments to those skilled in the art. The same reference numerals in the drawings represent the same or similar structures, so repeated descriptions of them will be omitted.

The present disclosure provides an anastomosis protection device, which includes a protective sleeve, a first fixing assembly, a second fixing assembly, and a power supply assembly. The protective sleeve is located inside the tubular tissue and positioned correspondingly to the anastomotic stoma. The protective sleeve protects an inner surface of the anastomotic stoma, so contents in the tubular tissue won't contaminate the anastomotic stoma when guided to pass through. The first fixing assembly is provided on an outer wall of the tubular tissue, and the first fixing assembly includes at least one electromagnetic member. The electromagnetic member produces a magnetic field when inputting a power supply current, so that the electromagnetic member becomes magnetic when powered on by the power supply assembly. The power supply assembly is electrically connected to the electromagnetic member for supplying power to the electromagnetic member. The second fixing assembly is provided on an inner surface of the protective sleeve. The second fixing assembly is a magnetic assembly or a non-magnetic assembly but can be attracted by magnetism, and is relatively fixed to the electromagnetic member by means of magnetic attraction. Therefore, the protective sleeve is fixed at a desired position through the magnetic attraction between the first fixing assembly and the second fixing assembly.

Since only the magnetic attraction position between the first fixing assembly and the second fixing assembly of the anastomosis protection device is relatively fixed, the device reduces impact on normal peristalsis of the tubular tissue and provides a circumferential extension and retraction space for the tubular tissue, ensuring normal blood supply around the anastomotic stoma. The first fixing assembly includes the electromagnetic member that produces a magnetic field to become magnetic when powered on. The magnetic field can be flexibly produced or eliminated by controlling on-off of the power supply, and a magnitude of magnetism in the electromagnetic member can be flexibly adjusted by adjusting a value of a power supply current and a number of turns on the electromagnetic member.

The following is a detailed structural introduction of the anastomosis protection device of each specific embodiment of the present disclosure, combined with the accompanying drawings. It can be understood that each specific embodiment is not a limitation of the protection scope of the present disclosure. In various embodiments, the intestinal tissue is used as an example for explanation. It can be understood that in other embodiments, the tubular tissue is not limited to intestinal tissue, but can also be other kinds of tubular tissues in the human body, such as tubular tissues at other positions in the digestive tract, all of which fall within the protection scope of the present disclosure.

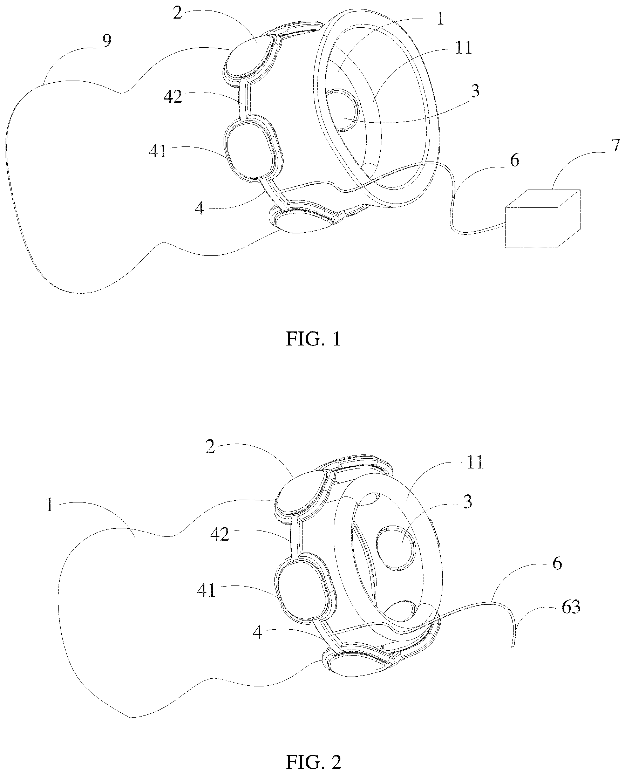

FIGS. 1-8 are structural schematic views of an anastomosis protection device applied to intestinal tissue 9 according to a first embodiment of the present disclosure. As shown in FIGS. 1-4, the anastomosis protection device includes: a protective sleeve 1 located inside the intestinal tissue 9 and positioned correspondingly to the anastomotic stoma, which protects an inner surface of the anastomotic stoma, so contents in the intestinal tissue 9 won't contaminate the anastomotic stoma when guided to pass through; a first fixing assembly provided on an outer wall of the intestinal tissue 9, and the first fixing assembly including at least one electromagnetic member; a power supply assembly electrically connected to the electromagnetic member, used for supplying power to the electromagnetic member, wherein the electromagnetic member is configured to become magnetic when powered on by the power supply assembly; and a second fixing assembly provided on an inner surface of the protective sleeve 1. The second fixing assembly and the electromagnetic member of the first fixing assembly are relatively fixed by means of magnetic attraction.

In the device, only the magnetic attraction position between the first fixing assembly and the second fixing assembly is relatively fixed, which reduces the impact on normal peristalsis of the tubular tissue and provides a circumferential extension and retraction space for the tubular tissue, ensuring normal blood supply around the anastomotic stoma. The electromagnetic member in the first fixing assembly produces a magnetic field to become magnetic when powered on by the power supply assembly. Therefore, the magnetic field can be flexibly produced or eliminated by controlling on-off of the power supply, and a magnitude of magnetism in the electromagnetic member can be flexibly adjusted by adjusting a value of the power supply current.

In this embodiment, the electromagnetic member includes at least one electromagnet 2. An electromagnet is a structure that produces a magnetic field to become magnetic when powered on, including a conductive winding wound outside an iron core, and a power of the conductive winding matches with that of the iron core. When inputting a current to the conductive winding, the winding becomes magnetic like a magnet. Therefore, in this embodiment, a magnitude of magnetism in the electromagnet 2 when inputting the current can also be adjusted by controlling number of turns on the electromagnet 2. In other alternative embodiments, the electromagnetic member can also adopt other structures, such as electromagnetic coils, which all fall within the protection scope of the present disclosure.

As shown in FIGS. 1-4, in this embodiment, the first fixing assembly includes a plurality of electromagnets 2 arranged at intervals, which are arranged sequentially along a circumferential direction of the intestinal tissue 9 to uniformly and stably fix the protective sleeve 1. The second fixing assembly includes a plurality of internal fixing member 3 in one-to-one correspondence to the electromagnets 2. The internal fixing members 3 can be made of non-magnetic materials that can be attracted by magnets, such as iron, nickel, cobalt, or other similar metals and their alloys. Alternatively, the internal fixing members 3 can be made of magnetic materials, such as magnets. In this embodiment, there are a plurality of electromagnets 2 arranged at intervals, and a plurality of internal fixing members 3 arranged at intervals. A number of the electromagnets 2 and a number of the internal fixing members 3 can also be selected according to variable requirements, and a one-to-one correspondence, one-to-many relationship, or many-to-one relationship can be formed between the electromagnets 2 and the internal fixing members 3, all of which fall within the protection scope of the present disclosure. In other alternative embodiments, there can be only one electromagnet 2 and/ or only one internal fixing member 3.

As shown in FIG. 2, the protective sleeve 1 is a tubular protective sleeve that can form circumferential protection for the anastomotic stoma. The protective sleeve 1 can be a thin-walled flexible film sleeve, such as a rubber film sleeve, a silicone film sleeve, etc., but the present disclosure is not limited to this. In other alternative embodiments, the protective sleeve 1 can also adopt other shapes, entirely or partially covering the inner wall of the anastomotic stoma, all of which fall within the protection scope of the present disclosure.

The fixing assembly is selectively arranged at an upstream side of the anastomotic stoma to fix the protective sleeve 1, so that the protective sleeve 1 won't move towards a downstream side of the anastomotic stoma and can keep protecting the anastomotic stoma. In the present disclosure, the upstream of the anastomotic stoma and the downstream are terms described as referring to a moving direction of the contents in the intestine, that is, the intestinal contents move from the upstream side of the anastomotic stoma to the downstream side of the anastomotic stoma. When the anastomosis protection device is applied to a lower position of the intestine, the protective sleeve 1 guides the feces through the intestine, moving from the upstream side of the anastomotic stoma to the downstream side of the anastomotic stoma.

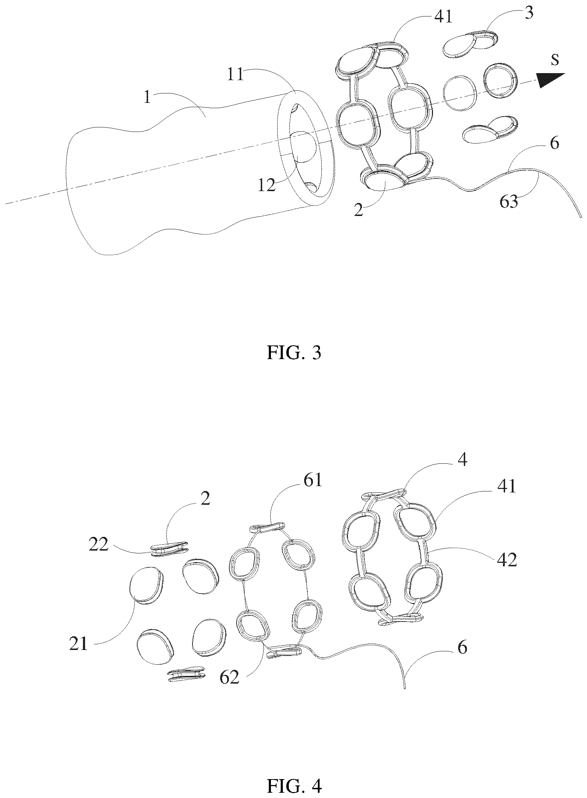

As shown in FIGS. 2 and 3, an end located at the upstream of the anastomotic stoma of the protective sleeve 1 is provided with a support portion 11, which is expandable and contractable in a radial direction of the protective sleeve 1. When the support portion 11 contracts in the radial direction, the protective sleeve 1 can be placed into or taken out of the intestinal tissue 9 as a whole. After the protective sleeve 1 is placed at the desired position, the support portion 11 expands in the radial direction to support the protective sleeve 1 and keep the protective sleeve 1 at the desired position. The support portion 11 can use an inflatable and deflatable circular airbag. The airbag is in a radial expansion state when inflated and in a radial contraction state when deflated. The support portion 11 can also use other structures, such as a radially expandable spring or a radially movable slider, which allow the support portion 11 to expand or contract radially. The first fixing assembly and the second fixing assembly are arranged between the anastomotic stoma and the support portion 11, and the protective sleeve 1 can be better maintained at the position corresponding to the anastomotic stoma under a combined action of the fixing assemblies and the support portion 11.

As shown in FIG. 1, in this embodiment, the power supply assembly includes a power supply wire 6, which surrounds the outer wall of the intestinal tissue 9 and is electrically connected to the electromagnet 2. Furthermore, the power supply assembly further includes an input power source 7. The power supply wire 6 can be electrically connected to the input power source 7, so the input power source 7 supplies power to the conductive winding of the electromagnet 2 through the power supply wire 6, causing the electromagnets 2 to become magnetic. The electromagnet 2 can produce a magnetic field when powered on and the produced magnetic field quickly disappears when powered off. When the electromagnets 2 need to be installed on the outer wall of intestinal tissue 9, the input power source 7 supplies power to the power supply wire 6, causing the electromagnets 2 to magnetically attach to the internal fixing members 3. During use, the power supply wire 6 continuously transmits power to the electromagnets 2. When the internal fixing members 3 are magnetic members, a magnetic polarity of the electromagnets 2 is opposite to a magnetic polarity of the internal fixing members 3. When the electromagnets 2 need to be removed from the outer wall of intestinal tissue 9, the power supply from the input power source 7 to the power supply wire 6 can be cut off, causing the electromagnets 2 to lose magnetism. By controlling the value of the power supply current in the power supply wire 6, the magnitude of the magnetism of the electromagnets 2 can also be changed. In other alternative embodiments, the electromagnets 2 can also adopt a structure that the produced magnetic field disappears slowly when powered off, that is, the electromagnets 2 can maintain magnetism for a period of time after being powered off.

As shown in FIG. 4, the power supply wire 6 includes a plurality of wire accommodating portions 61 and a plurality of wire connecting portions 62. The wire accommodating portion 61 is a circular hollow structure surrounding outer sides of the electromagnet 2, and the wire connecting portion 62 is connected between two adjacent wire accommodating portions 61. The wire accommodating portions 61 and the wire connecting portions 62 are combined to form a circular structure surrounding the outer wall of the intestinal tissue 9. As shown in FIG. 4, sides of the electromagnet 2 are provided with a first mounting groove 22 extending in a circumferential direction of the electromagnet 2, and the wire accommodating portion 61 of the power supply wire 6 is embedded in the first mounting groove 22, thereby stably connecting the electromagnet 2 with the power supply wire 6. In another alternative implementation, an inner side of the wire accommodating portion 61 of the power supply wire 6 is provided with a first mounting groove extending in a circumferential direction of the hollow structure of the wire accommodating portion 61, and the electromagnet 2 is embedded in the first mounting groove. To facilitate the installation of the electromagnets 2 on and removal from power supply wire 6, side walls of the electromagnets 2 adjacent to the inner wall of the installation hole have rounded corner structures 21.

In this embodiment, the power supply wire 6 is an elastic wire 6, and at least the wire connection portions 62 are elastic, allowing for elastic deformation with the intestinal peristalsis without applying limiting pressure, providing an extendable and retractable movement space for the intestine. When both the wire accommodating portions 61 and the wire connecting portions 62 are elastic, an elasticity of the wire connecting portion 62 is selectively greater than that of the wire accommodating portion 61. The wire accommodating portions 61 can be connected with the electromagnets 2 more stably, and elastic deformation capability of the wire connecting portions 62 is better, providing a greater circumferential extension and retraction space.

As shown in FIGS. 3-4, the power supply wire 6 also includes an extension portion 63, one end of the extension portion 63 is connected to the wire connection portions 62, and the other end of the extension portion 63 extends out of the body. The extension portion 63 can be electrically connected to the input power source 7.

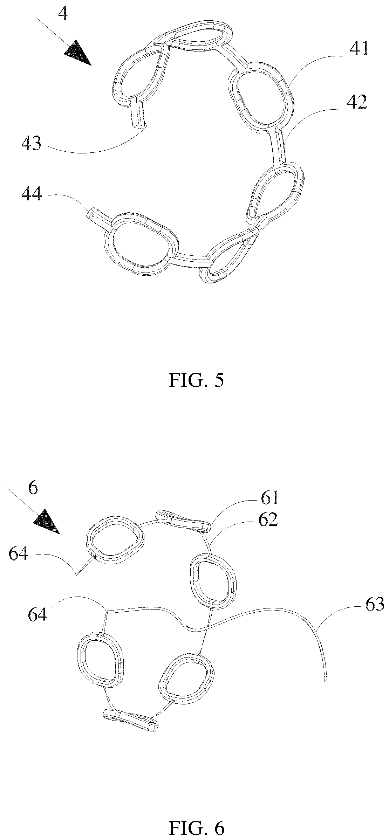

In this embodiment, to facilitate the installation and removal of power supply wire 6 at intestinal tissue 9, the power supply wire 6 includes a first connecting structure. The first connecting structure is a detachable connecting structure arranged at two ends of the power supply wire 6. Specifically, the power supply wire 6 is a strip-shaped wire having two ends, and the two ends thereof are provided with the detachable first connecting structure. For example, the first connecting structure includes a recess set at one end of the power supply wire 6 and a protrusion set at the other end of the power supply wire 6, the protrusion can be embedded in the recess to connect the two ends of the power supply wire 6. When the two ends are connected, the power supply wire 6 forms a closed ring. When the protrusion separates from the recess, the first connecting structure is disconnected, and the power supply wire 6 is back to the strip-shaped wire. In other alternative embodiments, the first connecting structure can also be a snap connecting structure, a threaded connecting structure, an adhesive connecting structure, etc. As shown in FIG. 6, when the power supply wire 6 is installed at the intestinal tissue 9, the power supply wire 6 can be connected at the first connecting structure to surround the outer wall of the intestinal tissue 9, for example, forming a closed circular structure. The circular structure of the power supply wire 6 can also be disconnected at two connection ends 64 of the power supply wire 6, making the power supply wire 6 an unclosed circular or strip-shaped structure that can separate from the outer wall of the intestinal tissue 9. The connection at the connection ends 64 of the power supply wire 6 can be achieved through snap connectors, hooks, bonding connectors, additional fixing parts, etc., all of which fall within the protection scope of the present disclosure. There are two implementation ways of the power supply wire 6: one is that an initial state of the power supply wire 6 is a closed ring, and when the power supply wire 6 needs to be removed from intestinal tissue 9, the first connecting structure is disconnected and the power supply wire 6 separates from the intestinal tissue 9; another way is an initial state of power supply wire 6 is an unclosed structure, after installed outside the intestinal tissue 9, and the power supply wire 6 is closed at the first connecting structure to form a ring.

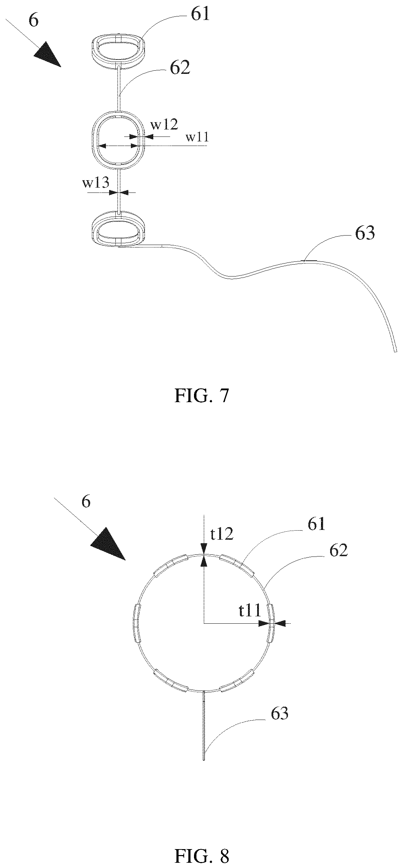

As shown in FIG. 7, a width w11 of the wire accommodating portion 61 can be greater than a width wl3 of the wire connecting portion 62. Therefore, the wire accommodating portions 61 can accommodate bigger electromagnets 2 to better fix the protective sleeve 1 with the electromagnets 2. The width of the wire connecting portions 62 is smaller, which can improve the elastic deformation capability of the wire connecting portions 62 and greatly reduce the impact on the peristalsis of the intestinal tissue 9. Furthermore, a width w12 of a circular wall of the wire accommodating portions 61 can be greater than the width w13 of the wire connecting portion 62, improving the connection stability between the wire accommodating portions 61 and the electromagnets 2. As shown in FIG. 8, a thickness t11 of the wire accommodating portion 61 can be substantially equal to a thickness t12 of the wire connecting portion 62. In another alternative implementation, the thickness t11 of the wire accommodating portion 61 can also be greater than the thickness t12 of the wire connecting portion 62 to accommodate thicker electromagnets 2 and further improve the elastic deformation capacity of the wire connecting portions 62, providing a greater circumferential extension and retraction space. The thickness here is described as referring to a radial direction of the power supply wire 6 after forming a circular structure (corresponding to the radial direction of the protective sleeve 1).

As shown in FIGS. 3 and 4, a surface of the electromagnet 2 facing the intestinal tissue 9 is an arc-shaped surface, and a shape of the arc-shaped surface is substantially adapted to the outer wall of intestinal tissue 9, to better fit the electromagnets 2 with the outer wall of intestinal tissue 9. A surface of the internal fixing member 3 facing the protective sleeve 1 is an arc-shaped surface, and a shape of the arc-shaped surface is substantially adapted to the inner surface of the protective sleeve 1, to better fit the internal fixing members 3 with the inner surface of the protective sleeve 1.

Furthermore, in another alternative embodiment, the surface of the electromagnet 2 facing the intestinal tissue 9 and/or the surface of the internal fixing member 3 facing the protective sleeve 1 may also be in a wavy shape. Specifically, the surface of the electromagnet 2 facing the intestinal tissue 9 is a wavy surface that ups and downs along a length direction of the intestinal tissue 9 (S direction as shown in FIG. 3), which can better fit the outer wall of intestinal tissue 9, increase the contact area between the electromagnets 2 and the intestinal tissue 9, and achieve better cooperation between the electromagnets 2 and the outer wall of the intestinal tissue 9. The surface of the internal fixing member 3 facing the protective sleeve 1 is a wavy surface that ups and downs along the length direction of the intestinal tissue 9 (S direction as shown in FIG. 3), which can increase the contact area between the internal fixing member 3 and the protective sleeve 1, and achieve better cooperation between the internal fixing members 3 and the inner wall of the protective sleeve 1.

In this embodiment, the first fixing assembly further includes a first connector 4 surrounding the outer wall of the intestinal tissue 9. The first connector 4 includes a plurality of first accommodating portions 41 being configured to accommodate the electromagnets 2 and a plurality of first connecting portions 42. The first connecting portion is connected between two adjacent first accommodating portions 41. The first accommodating portions 41 surround outer sides of the wire accommodating portions 61. The first connector 4 is elastic and can be elastically deformed along with the intestinal peristalsis, without applying limiting pressure on the intestine, providing an extendable and retractable movement space for the intestine. For example, the first connector 4 can be an elastic connector made of materials such as rubber, silicone, etc. with a certain elasticity. When both the first connecting portions 42 and the first accommodating portions 41 are elastic, an elasticity of the first accommodating portion 41 can be less than that of the first connecting portion 42. When a same tensile force is applied to the first accommodating portions 41 and the first connecting portions 42, an elastic deformation of the first accommodating portions 41 can be smaller than that of the first connecting portions 42. Therefore, the first accommodating portions 41 can better fix the electromagnets 2, and the first connecting portions 42 can provide better circumferential extension and retraction capacity.

As shown in FIG. 5, the first connector 4 is further provided with a second connecting structure, which can be connected at the second connecting structure to surround the outer wall of the intestinal tissue 9, or disconnected at the second connecting structure to be separated from the outer wall of the intestinal tissue 9. The second connecting structure shown in FIG. 5 includes a connecting protrusion 43 and a connecting recess 44 arranged at two ends, respectively. When the connecting protrusion 43 is embedded in the connecting recess 44, the first connector 4 is circular. When the connecting protrusion 43 separates from the connecting recess 44, the first connector 4 is in a strip shape or other unclosed shape. In other alternative embodiments, the connecting structure can also use other forms, such as a form including a connection ring and a connection hook at the two ends to achieve two states of the first connector 4 by hooking connecting ring to the connecting hook or separating them, or a form including a bonding structure at the two ends to achieve the two states of the first connector 4 through bonding or bonding separation, and so on.

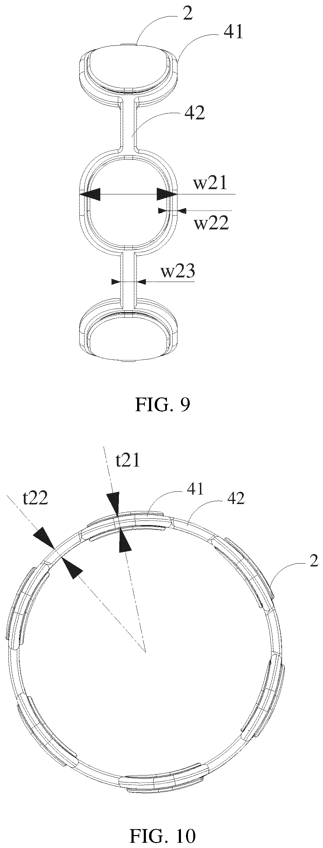

As shown in FIG. 9, a width w21 of the first accommodating portion 41 is greater than a width w23 of the first connecting portion 42. Therefore, the first accommodating portions 41 can accommodate bigger electromagnets 2 to better fix the protective sleeve 1 with the electromagnets 2. Moreover, the width of the first connecting portion 42 is smaller, which can improve the elastic deformation capability of the first connecting portions 42 and greatly reduce the impact on the peristalsis of the intestinal tissue 9. Furthermore, a width w22 of a circular wall of the first accommodating portion 41 can be greater than the width w23 of the first connecting portion 42, improving the connection stability between the first accommodating portions 41 and the electromagnets 2. In this embodiment, as shown in FIG. 10, a thickness t21 of the first accommodating portion 41 is substantially equal to a thickness t22 of the first connecting portion 42. In another alternative embodiment, the thickness t21 of the first accommodating portion 41 can also be greater than the thickness t22 of the first connecting portion 42. Therefore, the first accommodating portions 41 can accommodate thicker electromagnets 2 to better fix the protective sleeve 1 with the electromagnets 2. Moreover, the thickness of the first connecting portion 42 is relatively small, which can further improve the elastic deformation capability of the first connecting portions 42. The thickness here is described as referring to a radial direction of the first connector 4 after forming a circular structure (corresponding to the radial direction of the protective sleeve 1).

The first accommodating portions 41 are integrally formed with the first connecting portions 42, and the first accommodating portion 41 is a hollow circular structure surrounding the electromagnet 2, that is, an installation hole is formed in the first accommodating portion 41, and the electromagnet 2 is embedded in the installation hole. In other alternative embodiments, the first accommodating portions 41 can also adopt other shapes and can be independently formed and fixedly connected with the first connecting portions 42. As shown in FIG. 4, a circumferential first mounting groove 22 is provided on at least a part of side surfaces of the electromagnet 2, and the circular first accommodating portion 41 is embedded in the first mounting groove 22. In another alternative implementation, a circumferential mounting groove is provided on at least a part of an inner edge of the installation hole of the first accommodating portion 41, and the side surfaces of the electromagnet 2 are embedded in the mounting groove, thereby stably connecting the electromagnets 2 with the first accommodating portions 41. In this embodiment, the first accommodating portions 41 are located on the outer sides of the wire accommodating portions 61 of the power supply wire 6.

In the first embodiment and a third, fourth, fifth, sixth, seventh, ninth, and tenth embodiments described later, the first connector 4 can be partially or entirely made of bioabsorbable materials, such as bioabsorbable medical films, which have flexibility or elasticity while ensuring certain strength, thereby omitting the step of removing the first connector 4 after surgery.

As shown in FIG. 3, the inner surface of the protective sleeve 1 is further provided with a plurality of recesses in one-to-one correspondence with the internal fixing members 3, to better position the internal fixing members 3 on the inner surface of the protective sleeve 1. The internal fixing members 3 are embedded in the corresponding recesses 12, therefore, the internal fixing members 3 are detachably installed on the inner surface of the protective sleeve 1.

In the first embodiment and the second, third, fourth, seventh, eighth, ninth, and tenth embodiments described later, the internal fixing members 3 can be partially or entirely made of bioabsorbable materials, such as using bioabsorbable iron-based materials or magnetized bioabsorbable iron-based materials, thereby omitting the step of removing the internal fixing members 3 after surgery.

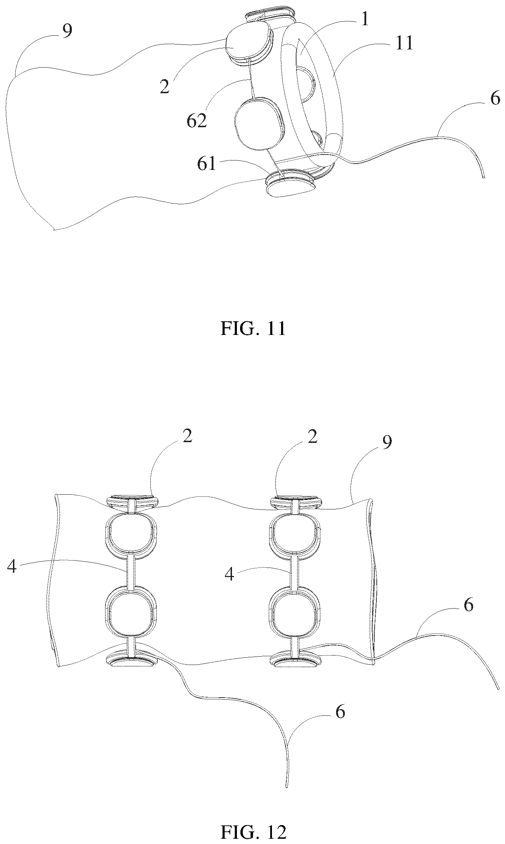

FIG. 11 is a structural schematic view of an anastomosis protection device applied to the intestinal tissue according to a second embodiment of the present disclosure. In this embodiment, there is no first connector in the first fixing assembly. The power supply wire 6 can simultaneously fix the electromagnets 2 and supply power to the electromagnets 2. The structure of the power supply wire 6, and the cooperation structure between the power supply wire 6 and the electromagnets 2 can adopt the specific structures in the first embodiment mentioned above, but the present disclosure is not limited to this. In other alternative embodiments, the power supply wire 6 can be designed as another structure and connected to the electromagnets 2 through other means, all of which fall within the protection scope of the present disclosure. The second fixing assembly can adopt the specific structure in the first embodiment mentioned above.

FIG. 12 is a structural schematic view of an anastomosis protection device applied to intestinal tissue according to a third embodiment of the present disclosure. In the third embodiment, the anastomosis protection device can be used for protecting the anastomotic stoma at a high position in the intestine. The anastomosis protection device includes two first fixing assemblies and two second fixing assemblies, one first fixing assembly and one second fixing assembly are set at the upstream side of the anastomotic stoma, and the other first fixing assembly and the other second fixing assembly are set on the downstream side of the anastomotic stoma. The first fixing assembly can adopt the structures of the first fixing assembly in the first and second embodiments, and the second fixing assembly can adopt the structures of the second fixing assembly in the first, fourth, fifth, or sixth embodiments. The structure of the first fixing assembly at the upstream side and the first fixing assembly at the downstream side can be the same with or different from each other, and the structure of the second fixing assembly at the upstream side and the downstream side can be the same with or different from each other. On the outer wall of intestinal tissue 9 at the upstream side of the anastomotic stoma, a set of electromagnets 2 is provided, and a set of internal fixing members 3 is correspondingly provided inside the protective sleeve 1. On the outer wall of intestinal tissue 9 at the downstream side of the anastomotic stoma, the other set of electromagnets 2 is provided, and the other set of internal fixing members is correspondingly provided inside the protective sleeve 1. This structure can greatly reduce a length of the required protective sleeve 1 and facilitate the installation of the protective sleeve 1 during surgery and the removal of the protective sleeve 1 after the anastomotic stoma is healed. On the other hand, it can better fix the protective sleeve 1 at the desired position, ensuring stability during use.

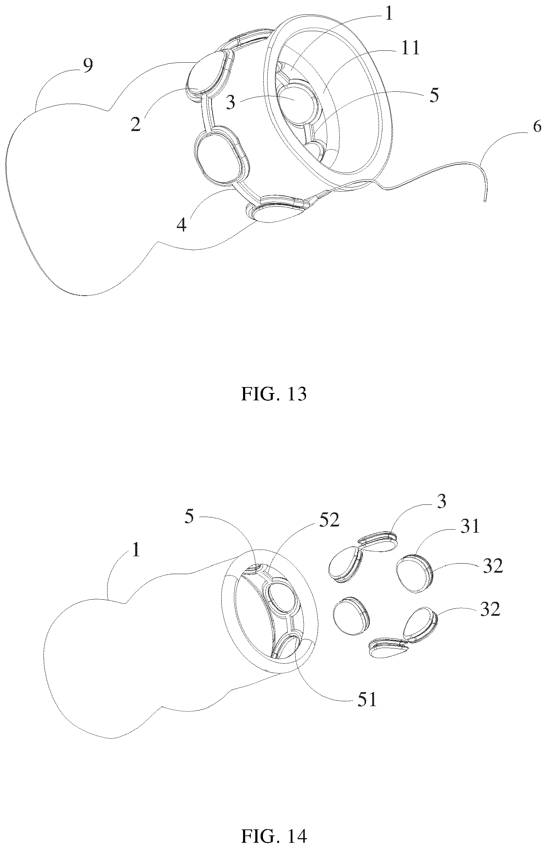

FIGS. 13-16 are structural schematic views of an anastomosis protection device applied to the intestinal tissue according to a fourth embodiment of the present disclosure. In this embodiment, the second fixing assembly further includes a second connector 5. When the second connector 5 is installed at the intestinal tissue 9, the second connector 5 surrounds the inner surface of the protective sleeve 1, forming a connecting ring structure, thereby fixing the internal fixing members 3 on the protective sleeve 1 in a circumferential direction. The second connector 5 is positioned correspondingly to the position of the first connector 4, forming a fixing ring for the protective sleeve 1. As shown in FIG. 14, the second connector 5 includes a plurality of second accommodating portions 51 and a plurality of second connecting portions 52. The second accommodating portions 51 are in one-to-one correspondence with the internal fixing members 3, and accommodate the corresponding internal fixing members 3. The second connecting portion 52 is connected between two adjacent second accommodating portions 51. When the second connector 5 is installed at the intestinal tissue 9, the second connector 5 is circumferentially extendable and retractable. Furthermore, the second connector 5 can be an elastic connector, and at least the second connecting portions 52 are elastic. Therefore, the second connector 5 can be elastically deformed along with the intestinal peristalsis, without applying limiting pressure on the intestine, providing an extendable and retractable movement space for the intestine. For example, the second connector 5 can be an elastic connector made of materials such as rubber, silicone, etc. with a certain elasticity. The second connector 5 can be arranged parallel to the support portion 11. The second connector 5 can be a closed circular structure or a connector having a connecting structure at two ends of the second connector 5. When installed at the intestinal tissue 9, two ends of the second connector 5 are connected to form a connecting ring. When both the second connecting portions 52 and the second accommodating portions 51 are elastic, an elasticity of the second accommodating portion 51 can be less than that of the second connecting portion 52. Thus, the second accommodating portions 51 can better fix the internal fixing members 3, and the second connecting portions 52 can provide better circumferential extension and retraction capacity.

Furthermore, in the third embodiment and a tenth embodiment described later, the second connector 5 can be partially or entirely made of bioabsorbable materials, such as using bioabsorbable medical films, which have flexibility or elasticity while ensuring certain strength, thereby omitting the step of removing the second connector 5 after surgery.

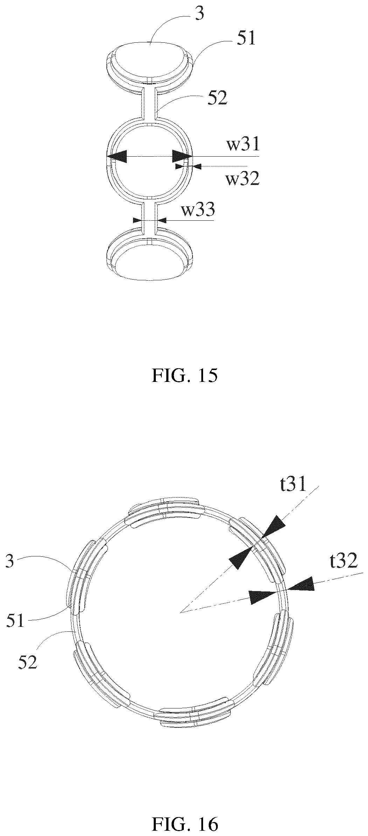

As shown in FIG. 15, in this embodiment, a width w31 of the second accommodating portion 51 is greater than a width w33 of the second connecting portion 52. Therefore, the second accommodating portions 51 can accommodate bigger internal fixing members 3 to better fix the protective sleeve 1 with the internal fixing members 3. Moreover, the width of the second connecting portion 52 is smaller, which can improve the elastic deformation capability of the second connecting portions 52 and greatly reduce the impact on the peristalsis of the intestinal tissue 9. Furthermore, a width w32 of a circular wall of the second accommodating portion 51 can be greater than the width w33 of the second connecting portion 42, thereby improving the connection stability between the second accommodating portions 51 and the internal fixing members 3. In this embodiment, as shown in FIG. 16, a thickness t31 of the second accommodating portion 51 is substantially equal to a thickness t32 of the second connecting portion 52. In another alternative embodiment, the thickness t31 of the second accommodating portion 51 can also be greater than the thickness t32 of the second connecting portion 52. Therefore, the second accommodating portions 51 can accommodate thicker internal fixing members 3, to better fix the protective sleeve 1 with the internal fixing members 3. Moreover, the thickness of the second connecting portion 52 is relatively small, which can further improve the elastic deformation capability of the second connecting portions 52.

As shown in FIG. 14, the second accommodating portions 51 are integrally formed with the second connecting portions 52, and the second accommodating portion 51 is a hollow circular structure surrounding the corresponding internal fixing member 3, that is, the second accommodating portion 51 is provided with an installation hole, and the internal fixing member 3 is embedded in the installation hole. To facilitate the installation of the internal fixing members 3 on the second connector 5 and the removal of the internal fixing members 3 from the second connector 5, the internal fixing member 3 has rounded corner structures 31 on side walls adjacent to an inner wall of the installation hole. In other alternative embodiments, the second accommodating portions 51 can also adopt other shapes and can be independently formed and fixedly connected with the second connecting portions 52. As shown in FIG. 14, a circumferential second mounting groove 32 is provided on at least a part of sides of the internal fixing member 3, and the circular second accommodating portion 51 is embedded in the second mounting groove 32. In another alternative implementation, an inner edge of the installation hole of the second accommodating portion 51 is at least partially provided with a second mounting groove in the circumferential direction of the installation hole, and the sides of the corresponding internal fixing member 3 are embedded in the second mounting groove, thereby stably connect the internal fixing members 3 with the second accommodating portions 51.

In this embodiment, the second connector 5 can also be integrally formed with the protective sleeve 1. In an alternative implementation, the second connector 5 can also be formed separately from the protective sleeve 1 and fixed to the protective sleeve 1 by adhesive or other means. In another alternative implementation, the second connector 5 can also be placed in the corresponding position of the protective sleeve 1 by a doctor during surgery, rather than being fixed to the protective sleeve 1 in advance.

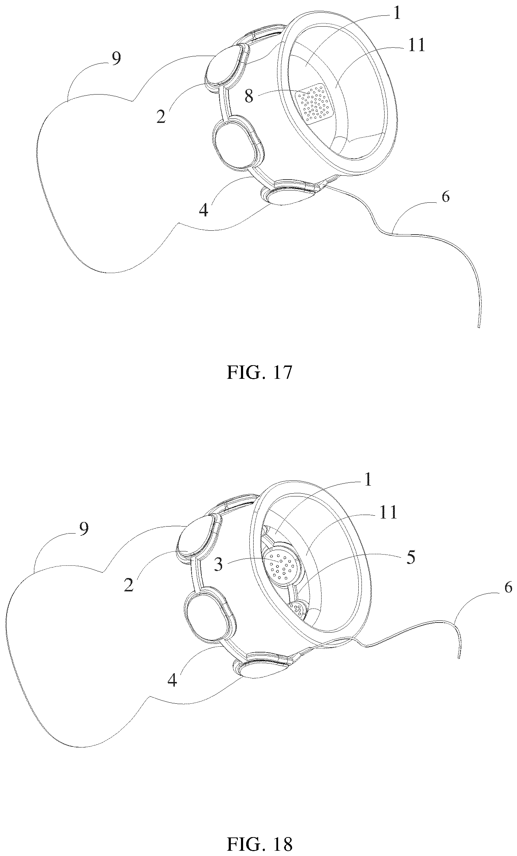

FIG. 17 is a structural schematic view of an anastomosis protection device applied to intestinal tissue according to a fifth embodiment of the present disclosure. In this embodiment, the second fixing assembly includes a plurality of magnetic particle coatings 8 coated on the inner surface of the protective sleeve 1, the magnetic particle coatings 8 are positioned correspondingly to the electromagnets 2, and a magnetic polarity of the magnetic particle coatings 8 is opposite to that of the electromagnets 2 when powered on. The magnetic particle coatings 8 can be formed by mixing magnetic particles with adhesives, solvents, etc., to form a magnetic slurry and then coating the magnetic slurry on the inner surface of the protective sleeve 1. Therefore, the second fixing assembly and the protective sleeve 1 form an integrated structure, which can be placed together at the desired position inside the intestinal tissue 9 during surgery. After the anastomotic stoma is healed, the second fixing assembly and the protective sleeve 1 can be removed together from the intestinal tissue 9. The structure of the second fixing assembly of the fifth embodiment can also be combined with the structure of the electromagnets 2 of each embodiment mentioned above. Similarly, by adjusting distribution density of magnetic particles and a total number of the magnetic particles, a magnetic force between the electromagnets 2 and the magnetic particle coatings 8 can be adjusted. Moreover, the magnetic particle coatings 8 are integrated with the protective sleeve 1, omitting the steps of installing the second fixing assembly onto and removing the second fixing assembly from the protective sleeve 1. Moreover, the second fixing assembly is in the form of magnetic particle coatings, occupying less space inside the protective sleeve 1.

FIG. 18 is a structural schematic view of an anastomosis protection device applied to intestinal tissue according to a sixth embodiment of the present disclosure. In this embodiment, at least a part of the surfaces of the internal fixing members 3 are coated with magnetic particle coatings or the internal fixing members 3 are internally doped with magnetic particles. The magnetic particles of the internal fixing member 3 have a same magnetic property, which is opposite to the magnetic property of the electromagnets 2.

In the fourth, fifth, and sixth embodiments mentioned above, the structure of the first fixing assembly, the structure of the power supply wire 6, and the cooperation structure between the first fixing assembly and the power supply wire can be the same as the structures in the first embodiment. In another alternative implementation, the first fixing assembly and/ or power supply wire 6 can also adopt other structures, and the cooperation structure between the first fixing assembly and the power supply wire can also adopt other structures.

After the fixing strap in the prior art surrounds the intestine, the pressure of the fixing strap to the intestine is fixed and cannot be adjusted. Based on this, the present disclosure further provides an anastomosis protection device, wherein a value of an output current of the power supply assembly is adjustable. The protective sleeve protecting the anastomotic stoma is fixed at the desired position through the magnetic attraction between the first fixing assembly and the second fixing assembly. The magnetic force generated by the first fixing assembly can be adjusted by using the power supply assembly with a regulable output current, thereby adjusting the pressure from the first and the second fixing assembles on the tubular tissue.

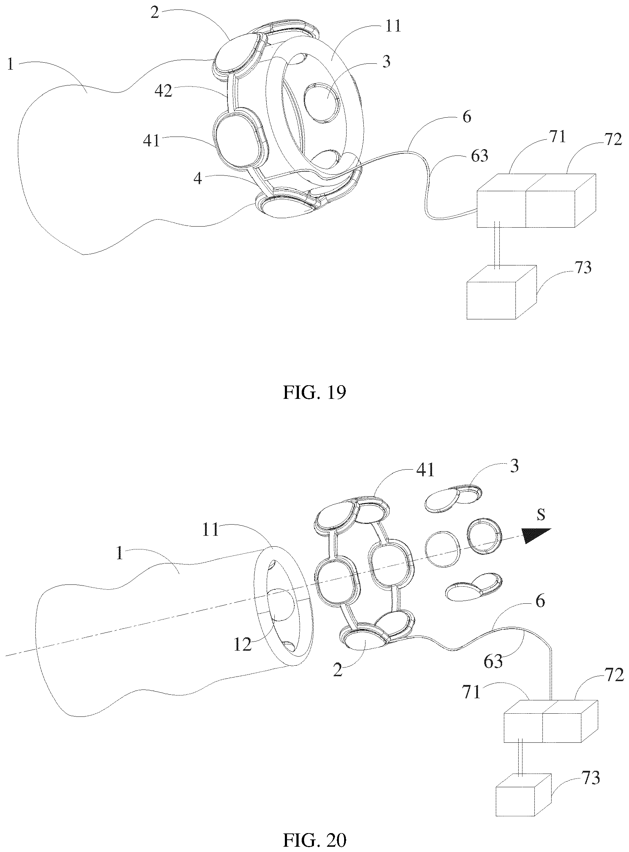

FIGS. 19-25 are structural schematic views of an anastomosis protection device according to a seventh embodiment of the present disclosure. The difference between the seventh embodiment and the first embodiment is that the structure of the power supply assembly is different. The anastomosis protection device includes: a protective sleeve 1 located inside the intestinal tissue 9 and positioned correspondingly to the anastomotic stoma; a first fixing assembly provided on the outer wall of the intestinal tissue 9, and the first fixing assembly including a plurality of electromagnetic members; a power supply assembly for supplying power to the electromagnetic members, wherein the electromagnetic members are configured to become magnetic when powered on, and an output current of the power supply assembly is regulable; and a second fixing assembly provided on the inner surface of the protective sleeve 1. The protective sleeve 1 protects the inner surface of the anastomotic stoma, so contents in the tubular tissue won't contaminate the anastomotic stoma when guided to pass through. The second fixing assembly and the electromagnetic members of the first fixing assembly are relatively fixed through the magnetic attraction.

Since only the magnetic attraction positions between the first fixing assembly and the second fixing assembly in the device are relatively fixed, the impact on the normal peristalsis of tubular tissue is reduced, providing a circumferential extension and retraction space for the tubular tissue, and ensuring normal blood supply around the anastomotic stoma. The electromagnetic members in the first fixing assembly can produce a magnetic field when powered on by the power supply assembly. Therefore, the magnetic field can be flexibly produced or eliminated by controlling on-off of the power supply. The magnetic force generated by the electromagnetic members can be adjusted by using the power supply assembly with the regulable output current, thereby adjusting the pressure from the first fixing assembly and the second fixing assembly on the tubular tissue.

In this embodiment, the electromagnetic members include electromagnets 2 as an example. In other alternative embodiments, the electromagnetic members can also adopt other structures, such as electromagnetic coils, which fall within the protection scope of the present disclosure.

As shown in FIGS. 19-21, in this embodiment, the first fixing assembly includes a plurality of electromagnets 2 arranged at intervals, which are arranged sequentially in the circumferential direction of the intestinal tissue 9 to uniformly and stably fix the protective sleeve 1. The second fixing assembly includes a plurality of internal fixing members 3 in one-to-one correspondence with the electromagnets 2. The internal fixing members 3 can be made of non-magnetic materials that can be attracted by magnets, such as iron, nickel, cobalt, or other metals and their alloys. Alternatively, the internal fixing members 3 can use a magnetic material with its own magnetism, such as a magnet, and a magnetic polarity of the internal fixing members 3 is opposite to that of the electromagnets 2 when powered on. In this embodiment, the electromagnets 2 are arranged at intervals, and the internal fixing members 3 are arranged at intervals. The number of electromagnets 2 and internal fixing members 3 can also be selected according to variable requirements, and a one-to-one correspondence, one-to-many relationship, or many-to-one relationship can be formed between the electromagnets 2 and the internal fixing members 3, all of which fall within the protection scope of the present disclosure. In other alternative embodiments, there can be only one electromagnet 2 and/ or only one internal fixing member 3.

As shown in FIG. 20, the protective sleeve 1 is a tubular protective sleeve that can form circumferential protection for the anastomotic stoma. The protective sleeve 1 can be a thin-walled flexible film sleeve, such as a rubber film sleeve, a silicone film sleeve, etc., but the present disclosure is not limited to this. In other alternative embodiments, the protective sleeve 1 can also adopt other shapes, entirely or partially covering the inner wall of the anastomotic stoma, all of which fall within the protection scope of the present disclosure. The fixing assembly is selectively arranged at the upstream side of the anastomotic stoma to fix the protective sleeve 1, so that the protective sleeve 1 won't move towards the downstream side of the anastomotic stoma and can keep protecting the anastomotic stoma.

As shown in FIGS. 19 and 20, an end located at the upstream of the anastomotic stoma of the protective sleeve 1 is provided with a support portion 11, which is expandable and contractable in a radial direction of the protective sleeve 1. The structure and working principle of the support portion 11 can be the same as that of the support portion 11 in the first embodiment mentioned above. The first fixing assembly and the second fixing assembly are arranged between the anastomotic stoma and the support portion 11, and the protective sleeve 1 can be better maintained at the position corresponding to the anastomotic stoma through cooperation between the fixing assemblies and the support portion 11.

As shown in FIG. 19, in this embodiment, the power supply includes a power supply wire 6, a current regulating circuit 71, a control element 73, and an input power source 72. The input power source 72 supplies power to the electromagnet 2 sequentially through the current regulating circuit 71 and the power supply wire 6, and the control element 73 is used to adjust a value of an output current of the current regulating circuit 71, The control element 73 can be a manual adjustment switch or an automatic control circuit, such as using a programmable logic controller (PLC). The input power source 72 can use one or more of rechargeable batteries or non-rechargeable batteries, etc. The current regulating circuit 71 can transmit an AC or DC current to the power supply wire 6. Correspondingly, the electromagnet 2 is an AC or DC electromagnet.

The power supply wire 6 surrounds the outer wall of the intestinal tissue 9 and is electrically connected to the electromagnet 2. The power supply wire 6 can be electrically connected to the input power source 72, which supplies power to the conductive winding of electromagnets 2 through the current regulating circuit 71 and the power supply wire 6, causing the electromagnet 2 to become magnetic. The electromagnet 2 can produce a magnetic field when powered on and the produced magnetic field quickly disappears when powered off. When the electromagnets 2 need to be installed on the outer wall of intestinal tissue 9, the input power source 7 supplies power to the power supply wire 6, causing the electromagnets 2 to magnetically attach to the internal fixing members 3. During use, the power supply wire 6 continuously transmits power to the electromagnets 2. When the internal fixing members 3 are magnetic members, a magnetic polarity of the electromagnets 2 is opposite to a magnetic polarity of the internal fixing members 3. When the electromagnets 2 need to be removed from the outer wall of intestinal tissue 9, the power supply from the input power source 7 to the power supply wire 6 can be cut off, causing electromagnet 2 to lose its magnetism. By adjusting the value of the power supply current in the power supply wire 6, the magnitude of the magnetism of the electromagnets 2 can be changed.

The current regulating circuit 71 can adopt various different circuit structures, which can output a regulable output current according to an input driving signal. For example, the current regulating circuit includes a transformer having a secondary winding with an adjustable number of turns. The input power source 72 inputs a current to a primary winding of the transformer, and the secondary winding of the transformer outputs a power supply current to the power supply wire 6. By adjusting the number of turns on the secondary winding of the transformer, the power supply current output from the transformer can be adjusted. For another example, the current regulating circuit includes a current dividing circuit, and at least one current dividing branch of the current dividing circuit includes a variable resistor. An output current of the current dividing circuit can be regulated by adjusting the resistance of the variable resistor.

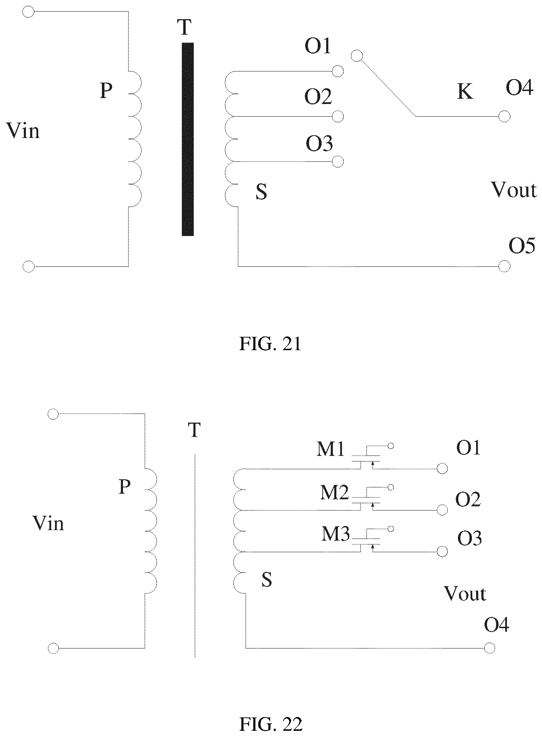

FIGS. 21 to 23 show structures of three kinds of current regulating circuit 71, which only exemplarily show the regulation principle of the current regulating circuit 71. In practical applications, the current regulating circuit 71 can include one or several structures of FIGS. 21 to 23, and/ or other required circuit components. For example, filtering circuits, voltage stabilizing circuits, current detection circuits, voltage detection circuits, etc. can be further added to the circuits in FIGS. 21 to 23.

FIG. 21 is a schematic view of the first kind of current regulating circuit in this embodiment. The current regulating circuit includes a transformer T, an input voltage Vin is input between two input terminals of a primary winding P of the transformer T, and an output voltage Vout is output between two output terminals O4 and O5 of a secondary winding S of the transformer T. The secondary winding S further has three output terminals O1, O2, and O3. A first end of a switch K has an adjustable contact, and the other end of the switch K is connected to the output terminal O4. By electrically connecting the adjustable contact of the switch K to different output terminals O1, O2, and O3, a voltage value of the output voltage Vout between the output terminals O4 and O5 can be adjusted. When the number of the turns on the electromagnets 2 remains unchanged, the change in the voltage Vout input to the electromagnets 2 changes a value of the power supply current transmitted from the current regulating circuit to the electromagnets 2. In this structure, the number of the turns on the secondary winding S has three adjustable values. In other embodiment, the number of the turns on the secondary winding S may only have two adjustable values or more than three adjustable values.

In this embodiment, the control element can be a manual switch or an automatic control circuit. For example, when the current regulating circuit includes a transformer T with adjustable turns on the secondary winding, the control element is a multi-position switch that can manually adjust the switch K. Each position of the multi-position switch corresponds to a position of the adjustable contact of the switch K. By adjusting the multi-position switch, the turns on the secondary winding of the transformer T can be adjusted. In another implementation, the control element includes a control circuit, which can generate a control signal to control the connection or disconnection between the adjustable contact of the switch K and different output terminals O1, O2, and O3, to adjust the turns on the secondary winding of the transformer T.

FIG. 22 is a schematic view of the second kind of current regulating circuit in this embodiment. Wherein, MOS (metal oxide semiconductor field effect) switches M1, M2, and M3 are provided between the secondary winding S of the transformer T and the three output terminals O1, O2, and O3, respectively. By controlling a gate voltage of the MOS switches separately, the connection between a source and a drain of each of the switches M1, M2, and M3 can be controlled, thereby achieving different output voltages Vout. For example, when only the source and drain of the switch M1 are connected, the output voltage Vout is the voltage between the output terminals O1 and O4. When only the source and drain of the switch M2 are connected, the output voltage Vout is the voltage between the output terminals O2 and O4. When only the source and drain of the switch M3 are connected, the output voltage Vout is the voltage between the output terminals O3 and O4. When the number of the turns on the electromagnets 2 remains unchanged, the change in the voltage Vout input to the electromagnets 2 changes the value of the power supply current transmitted from the current regulating circuit to the electromagnets 2. In this structure, the number of the turns on the secondary winding S has three adjustable values. In other embodiment, the number of the turns on the secondary winding S may only have two adjustable values or more than three adjustable values.

In this embodiment, the control element can be a manual switch or an automatic control circuit. For example, the control element can be a switch that can manually adjust the gate voltage of the switches, or a control circuit that can automatically adjust the gate voltage of the switches.

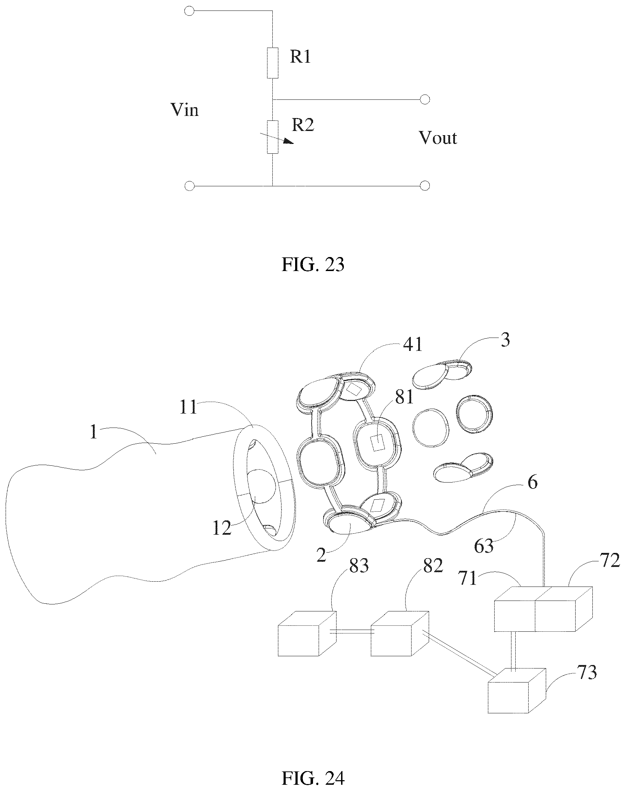

FIG. 23 is a schematic view of the third current regulating circuit in this embodiment. Wherein, a voltage dividing circuit is provided, including a resistor R1 and a variable resistor R2 connected in series. When adjusting the resistance of the variable resistor R2, the output voltage Vout is adjusted. When the number of the turns on the electromagnets 2 remains unchanged, the change in the voltage Vout input to the electromagnets 2 changes the value of the power supply current transmitted from the current regulating circuit to the electromagnets 2. In another alternative implementation, at least two resistors connected in parallel can also be used to form a current dividing circuit, wherein at least one resistor is a variable resistor. The output current can be regulated by adjusting a current value passing through the variable resistor.

In this embodiment, the control element can be a manual switch or an automatic control circuit. For example, the control element is a switch that can manually adjust the resistance of the variable resistor R2. By operating the switch, the resistance of the variable resistor R2 connected to the current regulating circuit can be adjusted. For another example, the control element can also be a control circuit that automatically adjusts the resistance of the variable resistor R2.

It can be understood that in the above embodiments, the current regulating circuit with an adjustable current or voltage at multiple steps (values) is described. In another example, a voltage regulating circuit with stepless voltage regulation, or a variable resistance circuit with stepless resistance regulation can also be used.

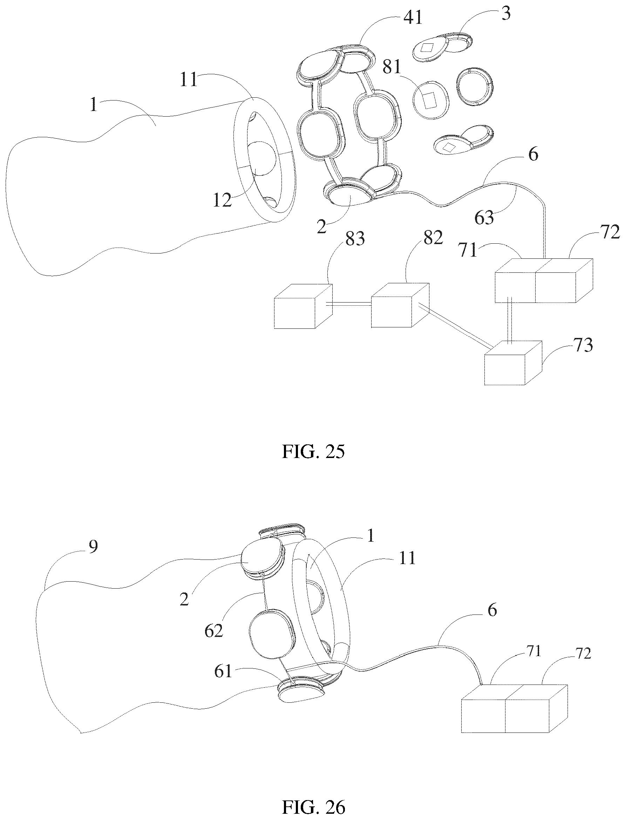

As shown in FIGS. 24 and 25, in this embodiment, the anastomosis protection device further includes a signal acquisition assembly, which includes a plurality of pressure sensor 81. The pressure sensors 81 are located at a side of the electromagnet 2 facing the tubular tissue and/or a side of the internal fixing member 3 facing the protective sleeve, or, the pressure sensors 81 are located on a side of the protective sleeve 1 facing the first fixing assembly or the second fixing assembly. FIG. 24 shows the structure of setting a plurality of pressure sensors 81 on the inner sides of the electromagnets 2. FIG. 25 shows the structure of setting a plurality of pressure sensors 81 on the outer sides of the internal fixing members 3. In another embodiment, the inner sides of some or all of the electromagnets 2 are provided with some pressure sensors 81, and the outer side of some or all of the internal fixing members 3 are also provided with some pressure sensors. The pressure sensors 81 can collect the pressure information between the electromagnets 2 and the internal fixing members 3, which corresponds to a magnitude of the magnetic force, thereby monitoring the magnetic force. The signal acquisition assembly can further include an A/D converter, which converts an analog signal collected by the pressure sensor 81 into a digital signal.

As shown in FIGS. 24 and 25, the anastomosis protection device further includes a signal processing assembly 82, which can adjust a power supply current value of the power supply assembly and then send a driving signal including the power supply current value to the power supply assembly. The power supply assembly 82 is configured to control the value of the output current based on the power supply current value. Specifically, the control element 73 includes a control circuit configured to control the output current automatically, and the driving signal is sent from the signal processing assembly 82 to the control element 73. The control element 73 can adjust the value of the output current of the current regulating circuit 71 based on the driving signal. For example, suppose the current regulating circuit 71 is in the form of FIG. 21, the control element 73 adjusts the adjustable contact of the switch K to be electrically connected to a corresponding output terminal of the output terminals O1, O2, or O3 based on a mapping relationship between variable power supply current values and the positions of the switch K, achieving regulable output current of the current regulating circuit 71. Suppose the current regulating circuit 71 is in the form of FIG. 22, the control element 73 controls the switches M1, M2, and M3 based on a mapping relationship between power supply current values and the switches. The gate voltages of the switches M1, M2 and M3 are adjusted to make the switches M1, M2, and M3 conductive or nonconductive, achieving regulable output current of the current regulating circuit 71. Suppose the current regulating circuit 71 is in the form of FIG. 23, the control element 73 adjusts the resistance of the variable resistor based on a mapping relationship between variable power supply current values and variable resistances, achieving regulable output current of the current regulating circuit 71.

The signal processing assembly 82 can adopt different methods when determining the power supply current value. For example, in one embodiment, the signal processing assembly 82 determines the power supply current value based on a predetermined power supply current change period, achieving periodic adjustment of the magnetic attraction between the electromagnets 2 and the internal fixing members 3, i.e., achieving periodic adjustment of the fixing force between the first fixing assembly and the second fixing assembly. In another implementation, the signal processing assembly 82 determines the power supply current value based on the detection data of the pressure sensors 81. For example, the signal processing assembly 82 compares the detection data of the pressure sensors 81 with a preset reference value. If the detection data of one pressure sensor 81 is smaller than the preset reference value, it indicates that the magnetic attraction is small and the power supply current value needs to be increased, and vice versa, if the detection data of one pressure sensor 81 is greater than the preset reference value, the power supply current value needs to be decreased. Furthermore, when a difference between the detection data of the pressure sensor 81 and the preset reference value exceeds a first preset threshold, the signal processing assembly can give an alarm, such as controlling an alarm to emit an alarm sound or light, or sending an alarm signal to a user terminal.

Furthermore, the signal processing assembly 82 compares the detection data of the pressure sensors 81 with the preset reference value, calculates an absolute value of the difference between the detection data of the pressure sensor 81 and the preset reference value, and determines whether the absolute value of the difference is greater than the first preset threshold. If so, it indicates that there is a significant difference between the actual detection data and the preset reference value, the signal processing assembly 82 needs to calculate a power supply current value based on the difference between the detection data of the pressure sensor 81 and the preset reference value, and send a driving signal including the power supply current value to the control element 73. If the absolute value of the difference is less than or equal to the preset first preset threshold, it indicates that the difference is within an acceptable range, and there is no need to calculate the power supply current value based on the difference. When there are a plurality of pressure sensors 81, the signal processing assembly 82 compares the detection data of a plurality of pressure sensors 81 with each other, and determines whether an error of the detection data of every two pressure sensors 81 is greater than a preset error threshold, and if so, the value of the output current of the current adjustment circuit 71 corresponding to one or more pressure sensors 81 needs to be adjusted, the signal processing assembly 82 generates a corresponding driving signal and sends the driving signal to the corresponding control element 73.

When there are a plurality of electromagnets 2 and a plurality of pressure sensors 81, a single current regulating circuit 71 can be used to supply power to a plurality of electromagnets 2, or a plurality of current regulating circuits 71 can be provided to supply power to the electromagnets 2 separately. When only one single current regulating circuit 71 is used, the signal processing assembly 82 determines the power supply current value based on the average detection data of the pressure sensors 81. Furthermore, when the error between every two pressure sensors 81 exceeds a second preset threshold, the signal processing assembly 82 can give an alarm, such as controlling the alarm to emit an alarm sound or light, or sending an alarm signal to the user terminal. When a plurality of current regulating circuits 71 are used to supply power to the electromagnets 2, the signal processing assembly 82 determines the power supply current value of the current regulating circuit 71 based on the detection data of the pressure sensor 81, and then outputs the driving signal to the corresponding current regulating circuit 71.

The signal processing assembly 82 can transmit data through signal wires between the pressure sensors 81 and the current regulating circuit 71 of the power supply assembly. The signal wires can be arranged around the outer wall of the intestinal tissue 9, or in other ways. In another implementation, the signal processing assembly 82, the pressure sensors 81 and the current regulating circuit 71 of the power supply assembly can also transmit data through wireless communication. For example, wireless communication modules are added at the pressure sensors 81 and the current regulating circuit 71. The signal processing assembly 82 can also communicate with the pressure sensors 81 through signal wires and the current regulating circuit 71 through wireless communication. Or the signal processing assembly 82 communicates with the pressure sensor 81 through wireless communication and communicates with the current regulating circuit 71 through a signal wire. The signal processing assembly 82 can be further connected to a display module 83, which can display the detection data, i.e., pressure values of the pressure sensors 81. The signal processing assembly 82 can be implemented using a processor chip, or the signal processing assembly 82 and the display module 83 can be implemented using a user terminal, such as a mobile phone, laptop, tablet, desktop, etc., or the display module 83 can be implemented using a separate display screen. The display module 83 and the signal processing assembly 82 can communicate through signal wires or wireless communication. The display module 83 can further display the power supply current values to show the control status of the power supply assembly in real time. Furthermore, when the signal processing assembly 82 alarms, the display module 83 can further display the alarm signal and information related to the alarm signal, such as the cause of the alarm, detection data of the pressure sensors during the alarm, and the position of the pressure sensors to which the alarm directs.

In the seventh embodiment, the power supply wire 6 includes a plurality of wire accommodating portions 61, a plurality of wire connecting portions 62, and an extension portion 63. The structure of the power supply wire 6, the cooperation structure between the power supply wire 6 and the electromagnets 2, the structure of the first connector 4, the cooperation structure between the power supply wire 6 and the first connector 4, the shape of the electromagnets 2, the shape of the internal fixing members 3, the cooperation structure between the internal fixing members 3 and the protective sleeve 1, the materials used for each component in the anastomosis protection device can be the same as those described in the first embodiment. For example, each component's structure and connection structure of the anastomosis protection device can adopt the structure and connection structure of the components shown in FIGS. 4-10.

In the seventh embodiment, the second fixing assembly can also be replaced with a structure including a plurality of magnetic particle coatings in the fifth embodiment, which can use the structure of the magnetic particle coating described in the fifth embodiment. The second fixing assembly can also be replaced with the structure of the internal fixing members including magnetic particles in the sixth embodiment mentioned above, the structure of which can be the same as the internal fixing members and magnetic particles in the sixth embodiment mentioned above.

FIG. 26 is a structural schematic view of an anastomosis protection device applied to intestinal tissue 9 according to an eighth embodiment of the present disclosure. The difference between the eighth embodiment and the seventh embodiment is that there is no first connector in the first fixing assembly. The power supply wire 6 can simultaneously fix the electromagnets 2 and supply power to the electromagnets 2. The cooperation structure between the power supply wire 6 and the electromagnets 2 and the structure of the power supply wire 6 can adopt the specific structures in the first embodiment mentioned above, but the present disclosure is not limited to this. In other alternative embodiments, the power supply wire 6 can be designed as other structures and connected to the electromagnet 2 through other means, all of which fall within the protection scope of the present disclosure. The second fixing assembly can adopt the specific structure in the first embodiment mentioned above.

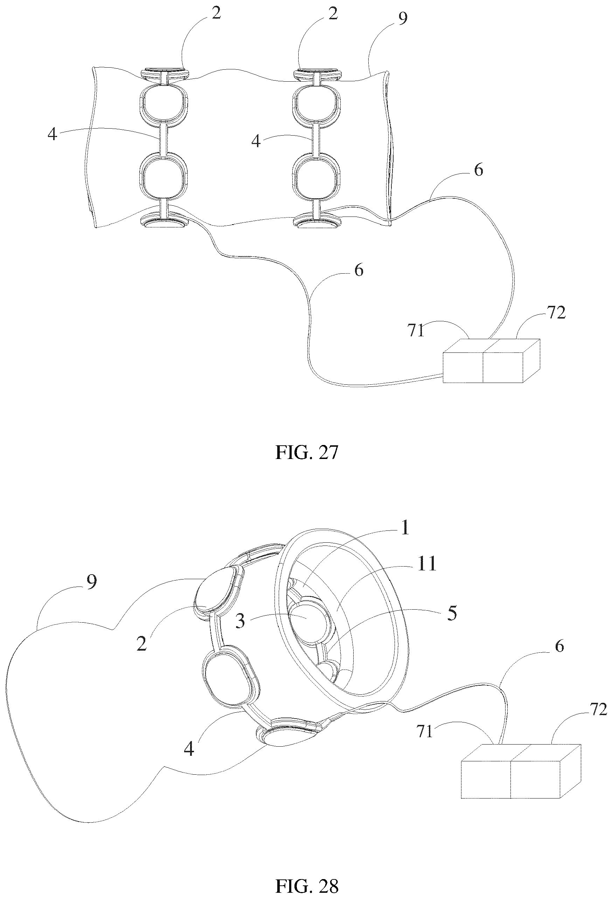

FIG. 27 is a structural schematic view of an anastomosis protection device applied to intestinal tissue 9 according to a ninth embodiment of the present disclosure. The difference between the ninth embodiment and the seventh embodiment is that the anastomosis protection device can be used for protecting the anastomotic stoma at a high position in the intestine. On the outer wall of intestinal tissue 9 at the upstream side of the anastomotic stoma, a set of electromagnets 2 is provided, and a set of internal fixing members is provided correspondingly inside the protective sleeve 1. On the outer wall of intestinal tissue 9 at the downstream side of the anastomotic stoma, another set of electromagnets 2 is provided, and another set of internal fixing members is provided correspondingly inside the protective sleeve 1. On the one hand, this structure can greatly decrease a length of the required protective sleeve 1. The protective sleeve 1 is only set at a desired position extending from the upstream to the downstream of the anastomotic stoma, and does not need to extend all the way to the anus; and it is more convenient to place the protective sleeve 1 during the surgical process and remove the protective sleeve 1 after the anastomotic stoma is healed. On the other hand, the two sets of fixing assemblies can better fix the protective sleeve 1 at the desired position, ensuring stability during use. The two sets of electromagnets 2 are connected through two power supply wires 6 to the power supply assembly, which can be simultaneously connected to a current regulating circuit 71 and an input power source 72. The magnetic forces of the two sets of electromagnets 2 can be adjusted through a current regulating circuit 71, or two current regulating circuits 71, respectively. In the ninth embodiment, the structure of the power supply wire 6, the cooperation structure between the power supply wire 6 and the electromagnets 2, and the structure of the first connector 4 can all adopt the structures of the first embodiment mentioned above.

FIG. 28 is a structural schematic view of an anastomosis protection device applied to intestinal tissue according to a tenth embodiment of the present disclosure. The difference between the tenth embodiment and the seventh embodiment is that the second fixing assembly further includes a second connector 5. When the second connector 5 is installed at the intestinal tissue 9, the second connector 5 surrounds the inner surface of the protective sleeve 1, forming a connecting ring structure, thereby fixing the protective sleeve 1 in a circumferential direction with the internal members 3. The second connector 5 is positioned correspondingly to the position of the first connector 4, forming a fixing ring for the protective sleeve 1. The structure of the second connector 5 of this embodiment and the cooperation structure between the second connector 5 and the internal fixing members 3 can be the same as the structures of the fourth embodiment mentioned above. Specifically, the structure shown in FIGS. 14-16 can be used. The structures of the first connector 4 and the electromagnets 2 in this embodiment can adopt the structures of the first embodiment mentioned above.

The materials of the anastomosis protection devices of different embodiments of the present disclosure are selectively biocompatible.

The anastomosis protection device provided by the present disclosure has the following advantages:
The device protects the inner surface of the anastomotic stoma through a protective sleeve, so contents in the tubular tissue won't contaminate the anastomotic stoma when passing through. The protective sleeve is fixed at the desired position through the magnetic attraction between the electromagnetic member of the first fixing assembly and the second fixing assembly. In this device, only the magnetic attraction position between the electromagnetic member and the second fixing assembly is relatively fixed, which reduces the impact on the normal peristalsis of the tubular tissue and provides a circumferential extension and retraction space for the tubular tissue, ensuring normal blood supply around the anastomotic stoma. By providing the electromagnetic member and the power supply assembly, the magnetic field can be flexibly produced or eliminated by controlling on-off of the power supply, and a magnitude of magnetism in the electromagnetic member can be flexibly adjusted by adjusting a value of the power supply current and a number of turns on the electromagnetic member. The anastomosis protection device of the present disclosure can be applied to the intestine or other tubular tissues in the human body, such as tubular tissues at other positions of the digestive tract.

The above is a detailed description of the present disclosure in connection with the specific preferred embodiments, and the specific embodiments of the present disclosure are not limited to the description. Modifications and substitutions can be made without departing from the spirit and scope of the present disclosure.

## Claims

1. An anastomosis protection device comprising:
a protective sleeve located inside a tubular tissue and positioned correspondingly to an anastomotic stoma;
a first fixing assembly provided on an outer wall of the tubular tissue, wherein the first fixing assembly comprises at least one electromagnetic member;
a power supply assembly electrically connected to the electromagnetic member, used for supplying power to the electromagnetic member, wherein the electromagnetic member is configured to become magnetic when powered on; and
a second fixing assembly provided on an inner surface of the protective sleeve, wherein the second fixing assembly and the electromagnetic member of the first fixing assembly are relatively fixed by means of magnetic attraction.

2. The device of claim 1, wherein the electromagnetic member comprises at least one electromagnet.

3. The device of claim 1, wherein the first fixing assembly comprises a plurality of electromagnetic members arranged at intervals, and the plurality of electromagnetic members are arranged sequentially in a circumferential direction of the tubular tissue.

4. The device of claim 3, wherein the second fixing assembly comprises a plurality of internal fixing members arranged at intervals, and the internal fixing members are magnetic members or attractable by magnets.

5. The device of claim 3, wherein the power supply assembly comprises a power supply wire, which surrounds the outer wall of the tubular tissue and is electrically connected to the electromagnetic members.

6. The device of claim 5, wherein the power supply wire comprises a plurality of wire accommodating portions and a plurality of wire connecting portions, the wire accommodating portions surround outer sides of the electromagnetic members, and the wire connecting portion is connected between two adjacent wire accommodating portions, the wire accommodating portions and the wire connecting portions together form a circular structure surrounding the outer wall of the tubular tissue.

7. The device of claim 6, wherein the power supply wire further comprises an extension portion, one end of the extension portion is connected to the wire connection portions, and the other end of the extension portion extends out of human body.

8. The device of claim 6, wherein an outer side of the electromagnetic member is provided with a first mounting groove in a circumferential direction of the electromagnetic member, and the wire accommodating portion of the power supply wire is embedded in the first mounting groove; or,
an inner side of the wire accommodating portion of the power supply wire is provided with a first mounting groove in a circumferential direction of the wire accommodating portion, and the electromagnetic member is embedded in the first mounting groove.

9. The device of claim 8, wherein a width of the wire accommodating portion is greater than a width of the wire connecting portion, and/or a thickness of the wire accommodating portion is greater than a thickness of the wire connecting portion.

10. The device of claim 6, wherein the power supply wire is elastic.

11. The device of claim 10, wherein an elasticity of the wire connecting portion is greater than an elasticity of the wire accommodating portion.

12. The device of claim 5, wherein the first fixing assembly further comprises a first connector surrounding the outer wall of the tubular tissue, the first connector comprises a plurality of first accommodating portions being configured to accommodate the electromagnetic members and a plurality of first connecting portions, the first connecting portion is connected between two adjacent first accommodating portions, the first accommodating portions surround outer sides of the wire accommodating portions.

13. The device of claim 5, wherein the power supply wire comprises a first connecting structure, the power supply wire has a state of being connected at the first connecting structure to surround the outer wall of the tubular tissue, and another state of being disconnected at the first connecting structure to be separated from the outer wall of the tubular tissue.

14. The device of claim 1, wherein the first fixing assembly further comprises a first connector surrounding the outer wall of the tubular tissue, and the first connector is connected to the electromagnetic member.

15. The device of claims 12 or 14, wherein the first connector comprises a second connecting structure, the first connector has a state of being connected at the second connecting structure to surround the outer wall of the tubular tissue, and another state of being disconnected at the second connecting structure to be separated from the outer wall of the tubular tissue.

16. The device of claim 14, wherein the first fixing component comprises a plurality of internal fixing members and a second connector being configured to accommodate the internal fixing members, and the second connector is located on the inner surface of the protective sleeve.

17. The device of claim 16, wherein at least a part of the first connector, the second connector, and/or the internal fixing members are made of bioabsorbable materials.

18. The device of claim 1, wherein the first fixing assembly further comprises a first connector being configured to accommodate the electromagnetic member; and/or the second fixing assembly comprises at least one internal fixing member and a second connector being configured to accommodate the internal fixing member;
at least a part of a circumference of the electromagnetic member is provided with a mounting groove, and an inner circumference of the first connector is embedded in the mounting groove, or at least a part of an inner circumference of the first connector is provided with at least one mounting groove, and the electromagnetic member is embedded in the mounting groove;
at least a part of a circumference of the internal fixing member is provided with a mounting groove, and an inner circumference of the second connector is embedded in the mounting groove, or at least a part of an inner circumference of the second connector is provided with at least one mounting groove, and the internal fixing member is embedded in the mounting groove.

19. The device of claim 1, wherein an end of the protective sleeve located at an upstream side of the anastomotic stoma is provided with a support portion, the support portion is expandable and contractable in a radial direction of the protective sleeve, and the first fixing assembly is arranged between the anastomotic stoma and the support portion.

20. The device of claim 1, wherein the first fixing assembly and the second fixing assembly are arranged at an upstream side of the anastomotic stoma; or
the anastomosis protection device comprises two first fixing assemblies and two second fixing assemblies, one of the first fixing assemblies and one of the second fixing assemblies are located at the upstream side of the anastomotic stoma, and the other first fixing assembly and the other second fixing assembly are located at the downstream side of the anastomotic stoma.

21. The device of claim 1, wherein an output current of the power supply assembly is regulable.

22. The device of claim 21, wherein the power supply assembly comprises an input power source, a current regulating circuit, a control element, and a power supply wire, the input power source supplies power to the electromagnetic member sequentially through the current regulating circuit and the power supply wire, and the control element is used for adjusting a value of an output current of the current regulating circuit.

23. The device of claim 22, wherein the current regulating circuit comprises a transformer, and a number of turns on a secondary winding of the transformer is adjustable, or
the current regulating circuit comprises a voltage dividing circuit or a current dividing circuit, and at least one current dividing branch in the voltage dividing circuit or the current dividing circuit comprises a variable resistor.

24. The device of claim 22, wherein the device further comprises a signal acquisition assembly, wherein the signal acquisition assembly comprises at least one pressure sensor arranged on a side of the first fixing assembly facing the tubular tissue and/or a side of the second fixing assembly facing the protective sleeve.

25. The device of claim 24, wherein the device further comprises a signal processing assembly used for determining a power supply current value based on a predetermined power supply current change period, and/or determining the power supply current value based on detection data of the pressure sensor;
the signal processing assembly is further used for sending a driving signal comprising the power supply current value to the power supply assembly, and the power supply assembly is configured to control a value of an output current based on the power supply current value.

26. The device of claim 25, wherein there are a plurality of pressor sensors, the power supply assembly comprises a plurality of electromagnetic driving modules provided correspondingly to the pressure sensors, and the signal processing assembly is configured to determine a power supply current value of the electromagnetic driving module based on the detection data of the corresponding pressure sensor, and send a driving signal comprising the power supply current value to the corresponding electromagnetic driving module.

27. The device of claim 25, wherein the signal processing assembly is further used for comparing the detection data of the pressure sensors with a preset reference value, and determining whether to alarm based on comparison results; or
the signal processing assembly is further used for comparing the detection data of a plurality of pressure sensors with each other, and determining whether to alarm based on comparison results; or
the signal processing assembly is further used for comparing the detection data of the pressure sensors with a preset reference value, or comparing the detection data of a plurality of pressure sensors with each other, and determining whether to generate the power supply current value based on comparison results, and send the driving signal comprising the power supply current value to the electromagnetic driving modules.

28. The device of claim 25, wherein the device further comprises a display module for displaying the detection data of the pressure sensor and/or a value of the output current of the power supply assembly.

29. The device of claim 25, wherein the signal processing assembly transmits data with the pressure sensor and the power supply assembly through signal wires or wireless communication, respectively.
